(19) Europäisches Patentamt
European Patent Office
Office européen des brevets

(11) Publication number: 0 246 854 A2

# (12) EUROPEAN PATENT APPLICATION

(21) Application number: 87304407.7

(51) Int. Cl.[4]: A61K 7/50

(22) Date of filing: 18.05.87

The title of the invention has been amended (Guidelines for Examination in the EPO, A-III, 7.3).

(30) Priority: 22.05.86 GB 8612550

(43) Date of publication of application: 25.11.87 Bulletin 87/48

(84) Designated Contracting States: AT BE CH DE ES FR GB GR IT LI LU NL SE

(71) Applicant: Lingner + Fischer GmbH
Hermannstr. 7
D-7580 Buehl (Baden)(DE)

(72) Inventor: Hermann, Claus
Rosenstrasse 10
D-7570 Baden-Baden(DE)
Inventor: Wieg, Renate
Franz-Hals Weg 5
D-4010 Hilden(DE)

(74) Representative: Dayneswood, Trevor et al
Beecham Pharmaceuticals Patent and Trade Mark Department Biosciences Research Centre Great Burgh Yew Tree Bottom Road
Epsom Surrey KT18 5XQ(GB)

## (54) Aqueous detergent shower gel composition.

(57) An aqueous detergent shower-gel composition comprises:

(a) 5-50% by weight of surfactant;

(b) 0.05-2% by weight of a guar gum derivative, especially guar hydroxypropyltrimethylammonium chloride;

(c) 0.5-10% by weight of hydrolysed animal protein, especially hydrolysed collagen;

(d) water; with a (b) : (c) weight ratio of 1:50 to 4:11.

EP 0 246 854 A2

## COMPOSITION

The present invention relates to a detergent composition, and in particular a composition for use as a shower gel.

Shower gel products are widely used, and most are in the form of detergent gels which can have a harsh effect on the skin.

A shower gel formulation has now been devised which produces a pleasant creamy feel on the skin of the user during application of the gel, and leaves the skin feeling less rough than with conventional gel formulations. The essence of the formulation lies in the surprising benefit obtained from using a mixture of a guar gum and a hydrolysed animal protein, the two components already being known for separate use in detergent formulations.

According to the present invention there is provided an aqueous detergent composition comprising:

(a) from 5 to 50% by weight of a surfactant,

(b) from 0.05 to 2% by weight of a guar gum derivative of the formula I

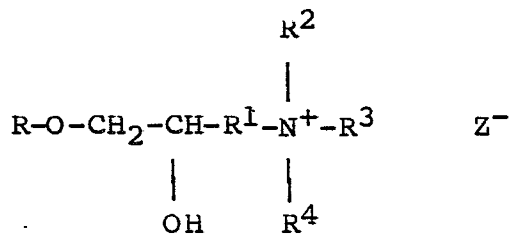

I

in which

R represents a guar gum radical,

$R^1$ represents a $C_{1-3}$ alkylene group,

each of $R^2$, $R^3$ and $R^4$ represents methyl, ethyl, hydroxyethyl or hydroxypropyl, and

$Z^-$ represents an anion,

(c) from 0.5 to 10% by weight of hydrolysed animal protein, and

(d) water, components (b) and (c) being present in weight ratio (b) : (c) of from 1:50 to 4:1.

In the formula I, $R^1$ preferably represents a methylene group, each of $R^2$, $R^3$ and $R^4$ preferably represents a methyl group, and $Z^-$ preferably represents $Cl^-$, $Br^-$, $I^-$ or $HSO_4^-$, especially $Cl^-$.

A particularly preferred guar gum derivative of the formula I for use as component (b) is guar hydroxypropyltrimethylammonium chloride, of the formula II

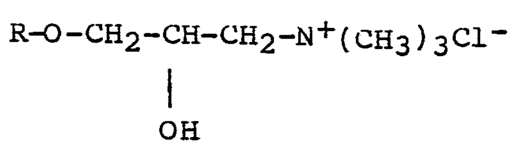

II

in which R represents a guar gum radical.

Such a material is commercially available from Henkel KgaA under the trade name 'Cosmedia Guar C261'.

The guar gum derivative, component (b), is preferably present in the composition of the invention in an amount of from 0.1 to 0.5% by weight. Components (b) and (c) are preferably present in a weight ratio (b) : (c) of from 1:10 to 2:1.

The hydrolysed animal protein used as component (c) is advantageously hydrolysed collagen, casein or elastin, preferably hydrolysed collagen. A particularly preferred material is commercially available from Henkel KGaA under the trade name 'Nutrilan L' (which is a hydrolysed collagen containing about 30% active material).

The hydrolysed animal protein, component (c), is preferably present in the composition of the invention in an amount of from 1 to 5% by weight.

The surfactant used as component (a) in the composition of the invention may be a cationic, anionic, nonionic or amphoteric surfactant or a combination of any of two or more thereof.

Examples of suitable anionic surfactants are sodium and ammonium lauryl sulphates, sodium and ammonium lauryl ether sulphates, sodium sulphonates, substituted ammonium lauryl sulphates, substituted ammonium lauryl ether sulphates, fatty acid alkanolamide sulphosuccinates, fatty acid sarcosinates, sodium lauryl ether sulphosuccinates, and magnesium lauryl ether carboxylates.

Examples of suitable nonionic surfactants are fatty acid alkanolamides, polyalkoxylated fatty acid amides, polyalkoxylated sorbitan esters of long chain fatty acids, polyalkoxylated long chain alkylamine oxides and amido amine oxides of long chain fatty acids, polyalkoxylated fatty alcohols, and glycerol esters of long chain fatty acids.

Examples of suitable cationic surfactants are polyethoxylated quaternary ammonium compounds, polyethoxylated fatty alkylamines, ethoxylated and quaternised condensation products of long chain fatty acids, and polyamines.

Examples of suitable amphoteric surfactants are betaines of the formula III

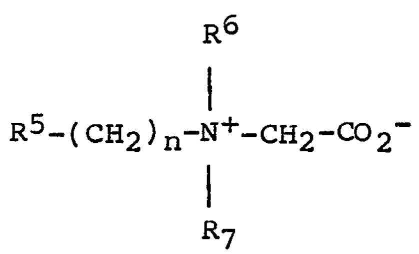

III

wherein

$R^5$ represents a $C_{7-13}$ alkyl group or a $C_{9-17}$ alkyl carbonylimino group;

each of $R^6$ and $R^7$ represents a $C_{1-3}$ alkyl group or a $C_{1-3}$ hydroxyalkyl group;

and n represents an integer from 1 to 5.

A particularly preferred betaine of the formula III is cocoamidopropylbetaine.

Other amphoteric surfactants which are suitable are those marketed under the trade name 'Miranol', and which have the formula IV

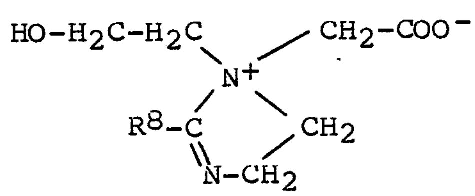

IV

in which $R^8$ represents an organic side chain.

A particularly preferred surfactant system comprises a blend of cationic and amphoteric surfactants, particularly a blend of an ether sulphate and a betaine surfactant, especially one of the formula III above. The most preferred blend comprises sodium lauryl ether sulphate and cocoamidopropylbetaine. The weight ratio of cationic to amphoteric surfactant perferably lies in the range of from 10:1 to 2:1.

The composition of the invention may also include minor amounts of other ingredients which are commonly employed in shower gel products, including, for example, essential oils, fatty oils, foam boosters, opacifiers, thickeners, perfumes, colouring agents, preservatives, and pH adjusting agents.

The compositions of the invention may be prepared by simple admixture of the various ingredients at ambient temperature. When an essential oil and/or a fatty oil is present, the guar gum is preferably mixed with the oil or oils prior to being admixed with the other ingredients, in order to obtain a homogeneous distribution of guar gum in the final blend.

The following examples illustrate compositions according to the invention. The words 'Nutrilan', 'Euperlan', 'Cosmedia', 'Softigen', 'Kathon', 'Glucamate', and 'Texapon' used in the examples are trade marks.

Example 1

A shower gel was prepared by mixing together the following ingredients in the given order:

| | | % w/w |
|---|---|---|
| water | | 57.55 |
| sodium lauryl ether-2 sulphate (56% by wt.) | | 25.00 |
| Nutrilan L (30%) (Henkel) (hydrolyzed animal protein from collagen) | | 2.50 |
| Euperlan PK 771 (Henkel) (sodium lauryl ether sulphate + glycol distearate + coconut monoethanolamide | | 1.50 |
| Cosmedia Guar C 261 (Henkel) (guar hydroxypropyltrimethylammonium chloride) | | 0.25 |
| perfume | 1.10 | |
| Softigen 767 (Dynamit Nobel) (PEG-6-caprylic/capric-glycerides) | 1.00 | |
| castor oil | 0.50 | |
| rape seed oil | 0.50 | |
| The above four components were premixed as a slurry | | 3.10 |
| Kathon CG (Rohm + Haas) (methylchloroisothiazolinone + methylisothiazolinone) | | 0.10 |
| cocoamidopropyl-betaine (30% by wt.) | | 10.00 |
| | | 100.00 |

Example 2

A shower gel was prepared by mixing together the following ingredients in the given order:

| | | % w/w |
|---|---|---|
| sodium myristyl ether sulphate | | 50.00 |
| cocoamphocarboxyglycinate (30%) | | 20.00 |
| hydrolyzed casein (50%) | | 3.00 |
| Glucamate DOE 120 (Nordmann + Rassmann) (PEG-120 methyl glucose dioleate) | | 0.75 |
| Euperlan PK 771 (Henkel) (sodium lauryl ether sulphate + glycol distearate + coconut monoethanolamide) | | 1.50 |
| Kathon CG (Rohm + Haas) (methylchloroisothiazolinone + methylisothiazolinone) | | 0.10 |
| Cosmedia Guar C 261 (Henkel) (guar hydroxypropyltrimethylammonium chloride) | | 0.50 |
| perfume | | 1.50 |
| Softigen 767 (Dynamit Nobel) (PEG-6-caprylic/capric glycerides) | | 0.30 |
| jojoba oil | | 0.05 |
| soybean oil | | 0.05 |
| Red 10 B 307002 (Food, Dyes and Colors Red No. 33; Colour Index No. 17200) | 0.00006 | |
| water (deionised) | 0.05994 | 0.06 |
| water | | 22.19 |
| | | 100.00 |

Example 3

A shower gel was prepared by mixing together the following ingredients in the given order:

| | | % w/w |
|---|---|---|
| Texapon ASV (Henkel) (mixture of sodium lauryl ether sulphate, magnesium lauryl ether sulphate, sodium lauryl ether-8 sulphate, magnesium lauryl ether-8 sulphate, sodium oleyl ether sulphate, + magnesium oleyl ether sulphate) | | 35.00 |
| coconut diethanolamide | | 2.00 |
| sodium lauryl ether-2 sulphate (56% by wt.) | | 10.00 |
| magnesium lauryl ether-10 carboxylate (35% by wt.) | | 12.50 |
| Nutrilan L (30%) (Henkel) (hydrolysed animal protein from collagen) | | 5.00 |
| Euperlan PK 771 (Henkel) (sodium lauryl ether sulphate + glycol distearate + coconut monoethanolamide) | | 1.50 |
| Kathon CG (Rohm + Haas) (methylchloroisothiazolinone + methylisothiazolinone) | | 0.10 |
| Cosmedia Guar C 261 (Henkel) (guar hydroxypropyltrimethylammonium chloride) | | 0.20 |
| almond oil | | 0.25 |
| peach oil | | 0.15 |
| perfume | | 2.00 |
| Red 10 B 307002 (Food, Dyes and Colors Red No. 33; Colour index No. 17200) | 0.00006 | |
| water (deionised) | 0.05994 | 0.06 |
| water | | 31.24 |
| | | 100.00 |

## Claims

1. An aqueous detergent composition comprising:
(a) from 5 to 50% by weight of a surfactant,
(b) from 0.05 to 2% by weight of a guar gum derivative of the formula I

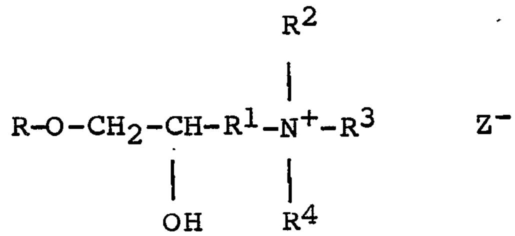

I

in which
R represents a guar gum radical,
$R^1$ represents a $C_{1-3}$ alkylene group,
each of $R^2$, $R^3$ and $R^4$ represents methyl , ethyl, hydroxyethyl or hydroxypropyl, and
$Z^-$ represents an anion,
(c) from 0.5 to 10% by weight of hydrolysed animal protein, and
(d) water,
components (b) and (c) being present in a weight ratio (b) : (c) of from 1:50 to 4:1.

2. A composition as claimed in claim 1, wherein component (b) comprises guar hydroxypropyltrimethylammonium chloride.

3. A composition as claimed in claim 1 or claim 2, wherein component (b) is present in an amount of from 0.1 to 0.5% by weight.

4. A composition as claimed in any one of claims 1 to 3, wherein components (b) and (c) are present in a weight ratio (b) : (c) of from 1:10 to 2:1.

5. A composition as claimed in any one of claims 1 to 4, wherein component (c) comprises hydrolysed collagen.

6. A composition as claimed in any one of claims 1 to 5, wherein component (c) is present in an amount of from 1 to 5% by weight.

7. A composition as claimed in any one of claims 1 to 6, wherein component (a) comprises an amphoteric surfactant.

8. A composition as claimed in claim 7, wherein the amphoteric surfactant is cocoamidopropylbetaine.

9. A composition as claimed in claim 7 or claim 8, wherein component (a) comprises a cationic surfactant and an amphoteric surfactant.

10. A composition as claimed in claim 9, wherein the weight ratio of cationic to amphoteric surfactant is from 10:1 to 2:1.

11. A process for preparing an aqueous detergent composition which comprises admixing the following compounds in any suitable order:
(a) from 5 to 50% by weight of a surfactant,
(b) from 0.05 to 2% by weight of a guar gum derivative of the formula I

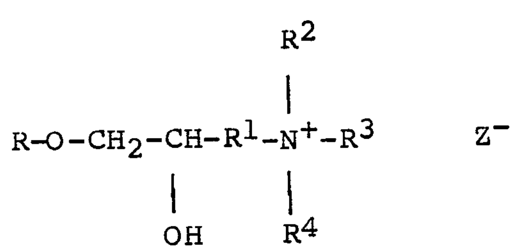

I

in which
R represents a guar gum radical,
$R^1$ represents a $C_{1-3}$ alkylene group,
each of $R^2$, $R^3$ and $R^4$ represents methyl, ethyl, hydroxyethyl or hydroxypropyl, and

$Z^-$ represents an anion,
(c) from 0.5 to 10% by weight of hydrolysed animal protein, and
(d) water,
components (b) and (c) being present in a weight ratio (b) : (c) of from 1:50 to 4:1.